Europäisches Patentamt

European Patent Office

Office européen des brevets

(19)

(11) **EP 1 374 764 A1**

(12) **EUROPEAN PATENT APPLICATION**
published in accordance with Art. 158(3) EPC

(43) Date of publication:
**02.01.2004 Bulletin 2004/01**

(51) Int Cl.⁷: **A61B 5/0295**, A61B 5/05

(21) Application number: **01989701.6**

(22) Date of filing: **27.12.2001**

(86) International application number:
**PCT/RU2001/000580**

(87) International publication number:
**WO 2002/054950 (18.07.2002 Gazette 2002/29)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **09.01.2001 RU 2001100643**

(71) Applicant: **Naumov, Valery Arkadievich
St. Petersburg, 193024 (RU)**

(72) Inventors:
• **NAUMOV, Valery Arkadievich
St.Petersburg, 193024 (RU)**
• **KLEVTSOV, Valery Alekseevich
St.Petersburg, 195299 (RU)**

(74) Representative:
**Reichert, Werner F. Dr. Dipl.-Phys.
Franz-Groedel-Strasse 1
61231 Bad Nauheim (DE)**

(54) **METHOD AND DEVICE FOR A RECORDING AND TREATING PLETHYSMOGRAM**

(57)    The invention relates to medical electronics and devices for testing the cardiovascular activity of human beings. The aim of the invention is to reduce energy consumption, lower costs and improve reliability, interference protection and repeatability of measurements. The inventive method consists in transforming a mechanical oscillation of the coating of a blood vessel into an electrical signal, amplifying and filtering the oscillation thus obtained, transforming it into a digital form by treating it in accordance with a specified program, The invention makes it possible to release a measured signal from a steady component and transform it into a pulse signal which is width-modulated by an amplified input signal. The width-modulated signal is transmitted to the input of a processor for treatment. The inventive device comprises a measured signal conditioner, an amplifier, a pulse-time modulator embodied for example in the form of a relaxation generator, and a processor of a computing system, all said elements being series connected.

**Figure 1**

**Description**

*Field of the Invention*

This invention relates to medical electronics and apparatus with integrated devices for testing the functioning of the cardiovascular system in the human being, including common household appliances.

*Background of the Invention*

It is well known that in Russian and in other counties diseases of the cardiovascular system prevail over others, and that they have a high death rate, being the cause of roughly 50% of deaths. At that, one of the fundamental difficulties in medicine is discovering abnormal functioning of the cardiovascular system in the early stages of heart disease.

Many methods and devices for measuring parameters of the cardio-vascular system (CVS) are known in the art, one of the most important and typical being heart contraction variability (HRV).

As is well known in the art, correct analysis of HRV gives practically enough information for preliminary assessment of the state of the CVS and for basic recommendations for further action by the patient to be made.

The invention is also related to methods and devices for measuring heart rhythm variability.

However, at this time, devices whose cost is within reach of the general public and that provide quick, accurate, and informative information of the state of the CVS are not known in the art. In solving this problem, the more interesting devices that exist in the art are devices that include specialized computer systems or other electronic systems that have as a component a processor or microprocessor and a device for displaying information, and that allow to make measurements of parameters of physiological processes taking place in a human body.

The rapid development of computer technology, along with widespread, pervasive use of powerful personal computers (PCs) and various devices containing microprocessors, has not touched enough upon using such systems for health monitoring in the household on a large scale.

A purpose of this invention is to eliminate the said deficiency and to improve upon known solutions to this technical problem.

Currently, the basic methods for measuring the parameters of the CVS known in the art are electrocardiography (ECG) and plethysmography (PG).

Electrocardiography is taking measurements of the electric field caused by electrical activity of the heart using electrodes placed on the skin for measuring the electric fields. Changes in voltage on the electrodes over time caused by the electric field are called the electrocardiogram. Taking measurements of CHANGES of blood volume due to heart contractions is called plethysmography. Changes in blood volume over time is called a plethysmogram. The simplest method for plethysmography is photoplethysmography (PPG), where light is directed onto an area of the skin so that it partially penetrates the tissue. This light is partially scattered and partially absorbed by the red blood cells. The light *reflected* by the skin is *converted* to an electrical signal by the photoelectric detector. The output signal of the photoelectric detector shows oscillations of the pulse due to blood volume changes caused by heart contractions. Another type of plethysmography known in the art is seismoplethysmography (SPG), where the blood volume changes are registered using a piezoelectric sensor.

Exemplary art where the above technical solutions are described include U.S. Pat. No. 3,980,075, U.S. Pat. No. 5,397,774, U.S. Pat. No. 5,423,322, and U.S. Pat. No. 5,632,272. However, none of these attempts in the art provide for direct links between the measuring device, placed on the patient, and peripheral devices of a PC.

Examples of the art most like the invention are described in U.S. Pat. No. 5,876,351 and U.S. Pat. No. 5,862,805.

U.S. Pat. No. 5,876,351 describes an autonomous device for taking electrocardiograms based on the NINTENDO GAMEBOY videogame. Using this videogame for taking electrocardiograms becomes possible by using a cassette containing all of the necessary electronic components and computer programs. Having a relatively high price ($50 US for the GAMEBOY and $125 US for the cassette), the device does not provide for a high level of diagnostic possibilities without changing the cassette, and also does not allow saving or statistically analyzing measured data.

A particular quality intrinsic to ECGs should be noted. It is that it is impossible to take electrocardiograms using a computer or a device (system) compatible with a computer without taking precautions for ensuring electrical safety. Indeed, when taking an ECG, the patient must be in electrical contact with the measuring device, which, in turn, must be connected with signal inputs of a computer. There is a galvanic coupling between the device and the computer, and so the patient can come to harm through electric shock. To exclude the possibility of injury from electric shock, an optoelectronic decoupler is used, and the power supply provided by a safe, separate, source. This raises the price of the measuring device. When taking electrocardiograms, it is necessary to take steps against interference, because a signal carrying information can have values in the range of fractions of a millivolt. A large disadvantage (for home use) of this method is that the patient must be prepared in order to take measurements. The preparation involves attaching electrodes onto the skin with preliminary application of electrically conducting lubricant, necessary for good contact between the skin and electrodes.

The PPG method is free of the said defects, as it does not need electrical contact between the patient and the computer and special preparation of the patient (except for ensuring that the temperature of the finger be above 28 degrees C).

U.S. Pat. No. 5,862,805 describes a method and device for measuring the variability of parameters of the CVS using PPG. The device includes a set of expensive electronic functional components: modulator, demodulator, analogue to digital converter (ADC) and processor for processing the measured signal. No link to a peripheral device of a PC is provided for. However, the general characteristics of this device and method is most like the invention. This patent is chosen as the prototype of the invention.

Generally, in other words, the method of this prototype is a method for registering and processing plethysmograms for measuring the parameters of the cardio-vascular system, providing for the plethysmogram to be taken by converting the oscillations of the walls blood vessels into an electric signal, then amplifying and filtering the resulting oscillations, then converting them into digital format and processing them using previously written programs giving conclusions and recommendations. At that, the possibility to output initial, intermediate, and final results of processing onto registering or displaying devices is allowed for. The plethysmogram is taken by illuminating the walls of the blood vessel with high frequency light and converting the reflected amplitude-modulated light into an electric signal that is further demodulated, filtered, and converted into digital format with further processing done in digital format.

The device of this prototype consists of an emitter of high frequency light, receiver for the reflected signal, demodulator, filter, analogue-digital converter and computer processor, connected in series.

The invention is fundamentally different in many aspects from prior art and the prototype. The fundamental difference is that a device executed in accordance with the invention can be used jointly with peripheral devices for input or output of information for computers or other electronic systems: the keyboard and mouse. The invention expands the functional possibilities of these devices. According to the invention, the device for measuring parameters of the CVS can be built into the case of the said peripheral devices. Furthermore, the serial port of the PC or other processing system, as well as being used for used for transmitting the measured signal into the PC or processing system, can also serve as the power source for the device, thus negating the need for an autonomous power supply. However, the serial ports of the PC have strict power restrictions, so the device implemented according to the invention does not include electronic components or units that have large power demands. Primarily, in prior art and in the prototype, the most power was used by the modulator, demodulator, ADC, data storage unit, and specialized processor. Along with large power demands, these functional units have a high price and lower the reliability of the device.

The said deficiencies in prior art and the prototype strongly limit their area of application and do not allow to solve the problem of supplying the general public (and, primarily, users of personal computer equipment and mobile telephones, and also users of personal and corporate vehicles) with devices whose price is within reach and that give timely and accurate information on the parameters of the CVS and that give anticipatory recommendations with the aim to stop the development of disease or anticipatory recommendations for further action for the user to take: to immediately visit a doctor or specialist, to stop using the computer or stop operating the vehicle, to take preventive measures, etc.

### *Summary of the Invention*

The main problem that the claimed invention aims to solve is to substantially lower the cost and power use, to improve the reliability, interference protection, accuracy, and repeatability of devices intended for registering and processing the parameters of the cardio-vascular system using plethysmography, in comparison with other devices known in the art (prior art and prototype).

The objects of the claimed invention are the method and device that provide the solution to the said problem.

In contrast with the most similar analogue - prototype (U.S. Pat. No. 5,862,805), which contains an autonomous power supply and the signal is processed by separate functional units that include a modulator, demodulator, ADC, and processor, and does not provide for a connection with an peripheral device of a host electronic system (HES) containing a processing unit and a device for displaying information, the processing and displaying of information from the measured signal, according to the claimed invention, is implemented with the help of the corresponding units of the HES (processor and device for displaying information); and, in addition, the ADC contained in the prototype is not used as the analogue measured signal is controlled by a relaxation generator (measuring generator unit) connected directly with the input/output signal port of the HES (for example, a PC), and the power for of all the peripheral units of the device (PUD) of the claimed invention offered to the consumer is provided by the input/output port of the said HES through a compulsory power stabilization unit.

The implementation of the described concept for building the device and the method for measuring the parameters of the CVS allow to transfer the functions of power supply, processing, and visualization of signals onto a powerful enough HES (for example, a PC), which allows to use low powered, safe electrical modes for implementing the processes that take place in the peripherals of the device, which make solving the problems associated with high accuracy and repeatability of the measurements much easier, and also lowers the cost of the PUD.

In addition to the already described set of elements of the present invention leading to the contemplated technical result, the set of elements of the claimed invention also comprises the below-described elements:

- the relaxation generator (RG) forms the bipolar, square-shaped pulses (in the "measuring generator" unit); at that, the pulses in one polarity (signal pulses) are related to the measured signal, while the pulses in the other polarity are the reference signal. The length of the signal pulses depend on the incoming voltage from the receiver. The transistor optoelectronic pair control the length of these pulses. This approach was chosen due to the necessity of decoupling the direct current between the high-gain direct-current amplifier (having a gain factor of 10,000, used to amplify the measured signal) and the RG. This way, any spurious coupling is eliminated, which improves stability (accuracy and repeatability of measurements);

- a unique feature of the invention is the effective way in which the influence of temperature, instabilities in the power supply, and other destabilizing factors are eliminated - the method of effectively correlating the length of the signal and reference pulses using program resources in the processor unit of the HES (for example, in the processor of a PC). The pulses are generated by the RG, so any destabilizing influence (temperature, humidity, instabilities in the power supply, etc.) affects the length of the signal and reference pulses equally. Usually, correlation in a wide range of lengths is a complex and expensive problem when using a signal processor. in the claimed invention, the processor unit of the HES receives both the signal and reference pulses, measures their lengths, and correlates them programmatically. The correlation value, which is used for any further processing, is related to the signal length without the influence of destabilizing factors;

- with the aim of providing a high stability for registration of the measured signal, the invention specifies that the signal be converted into a filtered series of packets (low frequency filtration) of signal pulses, which are then (the packets) processed in the processing unit of the HES (for example, a PC). For more accurate results, packets of pulses are processed instead of individual pulses. The essence of the filtration (or smoothing) method offered in the invention is in the accumulation of a defined number of registered points. At that, an array of the accumulated points is formed. The value of the first measurement is equal to the sum of the elements of the array. The next measurement value is defined by adding the next registered point to the array and simultaneously subtracting the first element of the array. At that, the new registered point replaces the subtracted element in the array.

The offered routine for low frequency filtering can be described by the following relationships:

$$S(1) = \sum_{i=1}^{n} M(i)$$

S(2)=S(1)-M(1)+t(n+1)
t(n+1)$\Rightarrow$M(1)
S(3) = S(2) - M(2) + t(n + 2)
t(n + 2) $\Rightarrow$M(2)
where S(1) first measurement value
i = 1K n- filter order
M(i) - array of accumulated registered points
S(2) - second measurement value
t(n + 1) - value of measurement n plus first registered point
t(n +1) $\Rightarrow$ M(1) - designates that the first point in the array is replaced with the n plus first registered point
S(3) - third measured value
t(n + 2) - value of measurement n plus second registered point

The replacement of elements of the array M(i) is done cyclically. After the last element in the array M(n) is replaced, the routine returns to the first element M(1). This method enables to perform smoothing of the signal without increasing the integration time, as the number of operations, three (subtraction, addition, and replacement), is always constant and independent of n. This enables the device to have the same time resolution independently of filtration order. The quantization time is equal to the period of the pulse train from the measuring generator. Thus, having taken measurements from a packet of signal pulses (stored in the memory of the PC), the low frequency filtration takes place practically without delay.

In the special case where a personal computer is to be used as a HES, the implementation requires that the computer be an IBM compatible with processor model 386 or higher and a free COM1 or COM2 port. The specifics of the invention functioning with a PUD built into a standard computer mouse connected with the said port of the PC, is that the measurement process must take place in real time, which means that the program must not be interrupted by other programs, that is, the computer must work in real mode. At that, the software of the PC, including the said program fitters, and executing all the operations of the claimed method in accordance with the structural diagram of the sequence of operations on the flowchart of the claimed device (figure attached), allows to produce, with high accuracy and repeatability, measurements of the parameters of the CVS, and to give expert analysis of the measurement results with conclusions on heart contraction rhythm disturbances. archiving (saving) of selected results, various types of monitoring, and also transmitting measurement results to a diagnostic center through the Internet and to receive corresponding recommendations from a specialist.

In other words, the claimed method for registering and processing plethysmograms for measuring the parameters of the cardio-vascular system, providing for taking the plethysmogram by converting the mechanical oscillations of the walls of a blood vessel into an electric signal, then amplifying and filtering the resultant oscillations, converting the oscillations into digital format, and processing them using previously written programs giving conclusions and recommendations, at that having taken into account the ability to output initial, intermediate, and final results onto displaying and recording equipment, specific in that it is capable of amplifying the electric signal, eliminate a constant component of the signal, and convert it into a pulse-length modulated (PLM) signal, modulated by the measured signal, that is then converted into digital format, and in digital format is filtered and processed in the processor of the computer, if necessary calibrating the measurement procedure against the variable and constant components of the PLM signal.

The device for implementing the claimed method includes connecting an initial measured signal generator in series with an amplifier, also using a PLM - digital converter and the processor of a computer. Another substantial difference is the introduction of a pulse-length modulator after the amplifier (the terminal signal generator - measuring generator unit); for example, a relaxation generator with one of the circuit arms connected to a resistor of an electronic-optical pair, of which the optical input is connected with the output of the amplifier. The output of the PLM modulator is connected with the processor, converting the PLM signal into a digital signal, which is then processed further after filtration.

### Brief Description of the Drawings

The flowchart diagram of the device of the invention.

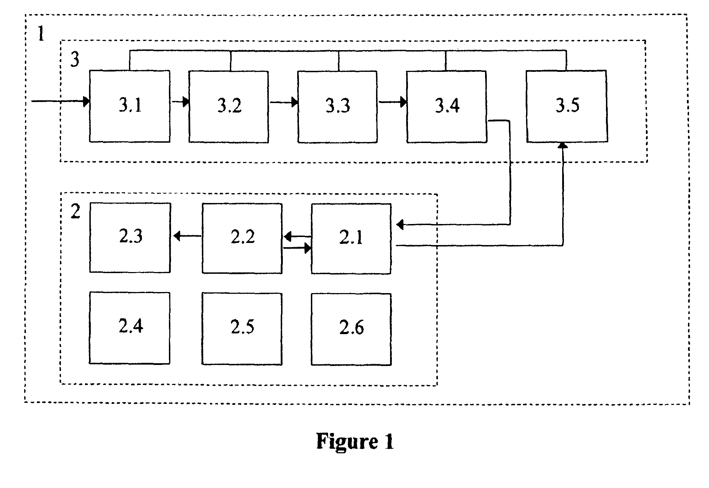

The figure shows the general case of the implementation of the invention; in particular, a personal computer can be used as the host electronic system, containing the processor, the input/output unit, and the device for displaying information.

The flowchart also illustrates which of the components are obligatory and the sequence of most of the steps of the operations that are necessary to perform when implementing the method according to the daimed invention.

### Information Supporting the Fact That the Invention Can Be Implemented

Possible implemented construction of the device according to the claimed invention is illustrated in the attached figures.

The figure is a flowchart diagram of the claimed invention (1), which includes units of the host electronic system (HES) (2), in particular, the input/output signal port (2.1 ), processor or microprocessor (2.2), and device for displaying information (2.3) (at that, the rest of the structural components of the HES (2), which for every type of HES will be different, are tentatively shown as 2.4, 2.5, 2.6), and the peripheral units of the claimed device (PUD) (3). including the initial measured signal generator (3.1) amplifiers and filters (3.2), the electronic-optical control unit (3.3), the terminal measured signal generator (measuring generator) (3.4) and the power supply stabilization unit (3.5). In accordance with the particularities of the invention mentioned herein, the PDU does not contain an ADC and an autonomous power supply, as the power for the structural units of the PDU (3) 3.1, 3.2, 3.3, and 3.4 is supplied by the input/output signal port 2.1 of the HES (2) through the power stabilization unit 3.5 of the PUD (3), and the output unit of the PUD (3) is directly connected with the input unit of the HES (2) - the input/output port 2.1.

The authors of the invention have constructed a device, fully compliant with the flowchart diagram in the included figure, where the PUD, built into a computer mouse by the firm "Genuis", has an infrared radiation source with wavelength approximately 0.9 micrometers, where the mouse is connected to COM 1 of an IBM compatible computer. The product set includes a diskette containing special software, 60 kB in volume, developed by the authors.

The electronic PUD is implemented using modem discrete components mounted on a circuit board using surface-mount technology. The choice of specific components to use was made from the point of view of optimal cost/value, The developed product is in pilot production at a specialized plant in St. Petersburg, Russian Federation. At this time, several batches of the product have been produced, and are sold to the consumer under the name "Dr. Mouse".

The case of the mouse has an opening through which the infrared radiation from the sensor leaves. When measuring arterial pulse, the patient covers the said opening with his/her palm of the right hand, in particular, with the bottom of the index finger near the base; when measuring capillary pulse, with the cushion of the index finger of the right hand.

Before taking measurements (strictly according to the instructions), the device must be ascertained to be in working condition: the output of PUD (3) is connected through the power stabilization unit 3.5 to the signal input/output port 2.1 of the personal computer 2; whereupon the power +/- 12 V, stabilized by the power stabilization unit 3.5, is supplied to all units of the PUD as illustrated by the figure: the initial measured signal generator 3.1, amplifier and filter 3.2, the electronic-optical control unit 3.3, the measuring generator unit 3.4, after which the supplied diskette is loaded by the computer, the directory DRMOUS is opened, and the program is run on the monitor of the computer 2.3, after which a view of "computer pulsometry" and a moving line appears, and the line moves up or down rapidly when the hand is moved in the vicinity of the sensor of the initial signal generator 3.1, after which the finger of the right hand (whose skin temperature is above 28 degrees Celsius) is used to close and slightly press against the opening in the case of the mouse where the opening in which the sensor is placed is located, and after 6 - 8 seconds the monitor 2.3 begins to display the pulse wave signal, and with the help of the up/down and left/right keys on the keyboard the amplitude and the placement of the pulse wave is adjusted, and when the behavior of the wave has stabilized the user enters the mode where the parameters of the CVS is measured: using the left hand, without moving the right hand, the user presses the F3 key (measurement) on the PC (2), after which, at moderate speed, the process of registration of the pulse will be displayed, at that, all operations take place according to the figure as specified by the claimed method: all structural elements of the PUD 3, without an autonomous power supply, are supplied with stabilized power through the power stabilizing unit 3.5 from the input/output signal port 2.1 of the PC 2, the primary measured signals are taken automatically from a finger, generated by the photodetector of the initial measured signal generator 3.1, amplified and filtered as signal pulses 3.2, then, through the electronic-optical control unit 3.3 are passed on to the measuringgenerator 3.4, where the signal and reference pulses of differing polarities are formed and passed to the processor 2.2 of the PC 2 through the input/output signal port 2.1, where, programmatically, the signal and reference signal pulse lengths are effectively correlated excluding the effects of destabilizing factors on the quality and value of the measured signals, and, at the same time, the processor 2.2 carries out, programmatically, filtration of the packets of signal pulses, their registration, and measurement of all parameters specified in the program; the user, without moving, for 12 - 15 seconds watches the process of registration taking place, during which the accumulation of results is taking place, after which the user presses the F4 key (display results), and the monitor displays the result of the measurements in tabular form, and when operated further, textual recommendations and clarifications for each parameter. The developed program allows to archive (save) the results, to perform various types of monitoring, to transmit data (when an Internet connection is available), through the Intemet to a Diagnostic Center and to receive answers - recommendations from specialists.

With the help of the Dr. Mouse product and the claimed method it is possible to measure and calculate the following parameters:

- pulse rate
- if there were any pauses or extrasystoles in a given time interval
- heart rhythm disturbances (arrhythmia)
- heart beat length variability
- pulse rate stability and variability

Analyzing the form of the pulse wave allows to determine:

- occurrence of vegetovascular dystonia
- disturbances in the functioning of the mitral and aortal valves
- weak contractions of the left ventricular heart muscles

The measurements have the following parameters:

- power supplied by COM 1, Volts        +12, -12
- CVS parameters measurement time, minutes          <=1
- measurement error, percent        +/- 5
- time resolution, milliseconds        10+/-2
- clock rate, kHz        1

Comparing the preproduction model of the Dr. Mouse device with the analogue - prototype, it can be seen that the power draw is lowered by a factor of 3, the number of electronic components is 2.5 times less, the cost is 7 - 8 times lower. In particular, the cost of the preproduction model (diskette + modified computer mouse) is $40-45 US. In mass production, the cost will be lower.

The Dr. Mouse device worked well with the "notebook" type PC - the area of application and consumer base grew substantially: with the help of such a system, consumers monitored themselves in various situations and environments: in gyms, in transportation, etc.

The products described above were on trial for long periods of time in the real-world environment in the city of St. Petersburg, Russian Federation, in special diagnostic points set up for this purpose in pharmacies, sport centers, and swimming pools. Overall, more than 10,000 people were tested. In all cases, the product has proved to be highly reliable, gave realistic results, and was accurate. Currently, the Dr. Mouse product is used in the first Intemet-pharmacies in the city of St. Petersburg, Russia, created with Pharmacy "Pharm-Baltic" (#66 Nevski Prospect) and Pharmacy No. 293 (#3 Prospect Bolshevikov).

At the same time, the device and method claimed by the authors as the invention is being actively tested by medical personnel in various medical facilities in the city of St. Petersburg. Russian Federation, for monitoring the status of the CVS in patients undergoing medical treatment, for monitoring the state of the heart and blood vessels after drug and alcohol intoxication, and in other extreme situations. After the corresponding statistical data has been collected, and the methodology has been formulated, the authors with present the necessary material for receiving a certificate from the Health Ministry of the Russian Federation.

The authors have done research lending support to the possibility of the PUD being implemented as a single chip (miniaturized technical solution) and the integration of the PUD into objects such as mobile (cellular) phones, compact units for testing the physiological state of a driver of a vehicle, digital video-cameras and still cameras, devices for controlling digital televisions, various biometric devices, including dactytoscopic devices, and other products of general and specialized use.

## Claims

1. The device and method for registering and processing plethysmograms for measuring the parameters of the cardio-vascular system, the device having an initial generator of the measured signal, and electronic amplifying circuit, terminal generator of the measured signal, processor device for processing the measured signal, a device for displaying information, and software, while the method presumes the use of software for processing the measured signal, calculating the variational characteristics of the functioning of the heart, and presenting the results of the processing and calculating on the screen of a device for displaying information

   wherein that in order to lower power use, to raise the reliability, to increase interference protection, accuracy, and, repeatability of the measurements of the parameters of the cardio-vascular system and to lower the cost of the device, a measuring generator of bipolar, square, measurement and reference pulses was used as a terminal signal generator instead of an analogue-digital converter, where the length of the measurement pulses depend on the voltage passed from the receiver of the initial signal generator, and the control over the length of the measurement pulses is realized by the transistor junction of the optoelectronic pair, after which the signal is passed from the measuring generator directly to the input/output signal port of the host electronic system, that together with the processor or microprocessor and the device for displaying information of the abovementioned host electronic system are included in the composition of the claimed device, and that the electric power for the units of the peripheral part: the initial signal generator, amplifier and generator, the electronic-optical control unit, and the terminal measured signal generator (measuring generator), is supplied through the power stabilization unit directly from the input/output signal port of the host electronic system, and using software, before the calculations on the parameters of the cardio-vascular system are performed, digital conversion and filtration of the measured signal from signal noise and instabilities in the power supply is performed.

2. Device and method according to claim 1 wherein the peripheral part of the device is embedded into the case of a computer mouse or computer keyboard.

3. Device and method according to claim 1 wherein the initial measured signal generator is implemented using a receiver and source of infrared radiation.

4. Device and method according to claim 1 wherein the initial measured signal generator is implemented using a receiver using the piezoelectric effect.

5. Device and method according to claim 3 wherein the receiver of infrared radiation does not directly contact the object of measurement, but remains hidden in the case, which can done by having a cavity on the inner side of the case without disturbing the integrity of the case and placing the receiver in the said cavity.

6. Device and method according to claim 1 wherein, with the aim of keeping the time resolution independent of the filtration order used, the filtration of the measured signal is done using the method of accumulating a given number of registration points, at that, the quantization time corresponds to the period of the pulse train of the measuring generator, in the beginning forming an array with a given number of registered points and the first measurement value is the sum of all the elements of the array, the next measurement value is defined by adding the value of the next registered point to the said sum and simultaneously subtracting the first element of the array, at that the new values of registered points replace the subtracted points and after the last element the routine returns to first element of the array.

7. Device and method according to claim 1 wherein the device is completely or partially embedded into the case of a digital video camera.

8. Device and method according to claim 1 wherein the device is completely or partially embedded into the case of a digital still camera.

9. Device and method according to claim 1 wherein the device is completely or partially embedded into the case of and external units of a digital television.

10. Device and method according to claim 1 wherein the device is completely or partially embedded into the case of mobile (cellular) phone.

11. Device and method according to claim 1 wherein the device is completely or partially included in a device intended for biometrics.

12. Device and method according to claim 1 wherein the device is completely or partially included in a device for taking and processing daktyloscopic data.

**Figure 1**

## INTERNATIONAL SEARCH REPORT

| | International application No. |
| --- | --- |
| | PCT/RU 01/00580 |

**A. CLASSIFICATION OF SUBJECT MATTER**
A61B 5/0295, 5/05

According to International Patent Classification (IPC) or to both national classification and IPC

**B. FIELDS SEARCHED**

Minimum documentation searched (classification system followed by classification symbols)
A61B 5/00, 5/02, 5/0295, 5/05-5/053

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | US 5862805 A (OPTELMED LTD.) 26 January 1999 (26.01.1999) | 1-12 |
| A | US 5876351 A (MITCHELL M. ROHDE) 02 March 1999 (02.03.1999) | 1-12 |
| A | RU 2054884 (MEZHOTRASLEVOI NAUCHNO- TEKNICHESKY KOMPLEKS "MIKROKHIRURGLIJA GLAZA") 27 February 1996 (27.02.1996) | 1-12 |
| A | SU 1521454 (INSTITUT TEKHNICHESKOI KIBERNETIKI AN BSSR et al.) 15 November 1989 (15.11.1989) | 1-12 |

☐ Further documents are listed in the continuation of Box C.    ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
| --- | --- |
| 25 March 2002 (25.03.2002) | 11 April 2002 (11.04.2002) |

| Name and mailing address of the ISA/ | Authorized officer |
| --- | --- |
| Facsimile No. | Telephone No. |

Form PCT/ISA/210 (second sheet) (July 1992)